# EUROPEAN PATENT SPECIFICATION

(11) **EP 4 384 147 B1**
(45) Date of publication and mention of the grant of the patent: **25.11.2026**
(21) Application number: 22856433.2
(22) Date of filing: 04.08.2022
(51) Int. Cl.: A61K 9/48, A61K 9/00, A61M 31/00

(54) **INGESTIBLE DEVICE**
EINNEHMBARE VORRICHTUNG
DISPOSITIF INGÉRABLE

(30) Priority: 09.08.2021 US 202163231206 P
(43) Date of publication of application: 19.06.2024
(73) Proprietor: Massachusetts Institute of Technology, Cambridge, MA 02139 (US); The Brigham and Women's Hospital, Inc., Boston, MA 02115 (US); Novo Nordisk A/S, 2880 Bagsvaerd (DK)
(72) Inventor: TRAVERSO, Carlo Giovanni, Newton, MA 02459 (US); GWYNNE, Declan, New York, NY 10032 (US); WAINER, Jacob, Malden, MA 02148 (US); ARRICK, Graham, San Francisco, CA 94110 (US); WINDUM, Jesper, 3400 Hillerod (DK); SANGER, Kuldeep, Kurukshetra, 136135 (IN); BOHR, Adam, 2450 Copenhagen (DK); MOURIDSEN, Brian, 2880 Bagsvaerd (DK); JAKOBSEN, Nikolaj Eusebius, 2860 Søborg (DK); FREDERIKSEN, Morten Revsgaard, 1424 Copenhagen (DK); LAURSEN, Jens Lolle, 4040 Jyllinge (DK); AGUILAR, Ludwig Erik, 3400 Hillerød (DK)
(74) Representative: Hoffmann Eitle
(86) International application number: PCT/US2022/039374
(87) International publication number: WO 2023/018591

(56) References cited:
- WO-A1-2022/055779
- US-A1- 2010 021 536
- US-A1- 2012 010 590
- US-A1- 2012 165 793
- US-A1- 2018 250 226
- US-A1- 2020 129 441

## Description

### FIELD

Disclosed embodiments relate to ingestible devices, for example, devices that deploy from a first state to a second state in the gastrointestinal tract of a subject.

### BACKGROUND

Typically, interacting with the gastrointestinal (GI) tract of a subject may pose particular challenges. For example, certain therapeutics are composed of large and complex molecules that denature readily when administered via the oral-gastrointestinal (GI) route. Accordingly, subjects who need these therapeutics typically use more invasive forms of drug administration that are outside the GI route including, for example, subcutaneous injection.

In addition to the above, uptake of hormonal therapies remains limited due to an inability to administer them orally. Specifically, ingested hormones may be broken down in the gastrointestinal tract, prior to uptake. Correspondingly, hormones are typically injected. This significantly reduces the adherence to hormonal therapies and limits subject compliance. Electroceutical therapies is an emerging approach that uses electrical stimulation of various tissues to affect the metabolism of a subject.

WO 2022/055779 A1 relates to a swallowable drug delivery device including a capsule and a tissue attachment member.US 2012/165793 A1 relates to devices and related methods for the controlled delivery of a therapeutic to a targeted location within a body.

US 2010/021536 A1 relates to an ingestible capsule for delivering medication to a subject.

### BRIEF SUMMARY

The present invention is defined by the appended independent claims. The dependent claims are directed to optional features and preferred embodiments.

In aspects of the present invention, there are provided ingestible devices according to the independent claims.

Preferred embodiments are set out in the dependent claims and in the remaining part of the description.

Associated methods are also described herein to aid understanding of the invention, but these do not form part of the claimed invention.

In one embodiment an ingestible device comprising: a body; at least one limb is configured to deploy from a first configuration to a second configuration after exposure to a predetermined condition within a gastrointestinal tract of a subject, wherein in the first configuration the at least one limb is wrapped around at least a portion of an outer surface of the body, and wherein in the second configuration the limb extends outward from the body; and an active portion of the ingestible device disposed on an exterior portion of the body and/or the at least one limb, wherein the at least one limb is configured to orient the body such that the active portion contacts an adjacent tissue surface underlying the body relative to a direction of gravity when the at least one limb is in the second configuration, and wherein the active portion is configured to interact with the adjacent tissue surface.

In a second embodiment an ingestible device comprising: a body; at least one limbis configured to deploy from a first configuration to a second configuration after exposure to a predetermined condition within a gastrointestinal tract of a subject, wherein in the first configuration the at least one limb is wrapped around at least a portion of an outer surface of the body, and wherein in the second configuration the limb extends outward from the body; and an active portion of the ingestible device disposed on an exterior portion of the body and/or the at least one limb, wherein the at least one limb is configured to orient the body such that the active portion contacts an adjacent tissue surface underlying the body relative to a direction of gravity when the at least one limb is in the second configuration, and wherein the active portion is configured to interact with the adjacent tissue surface.

A not claimed method of orienting an ingestible device is disclosed. The method includes exposing the ingestible device to a predetermined condition within a gastrointestinal tract of a subject; deploying at least one limb attached to a body of the ingestible device such that the at least one limb transitions from a first configuration to a second configuration after exposure to the predetermined condition, wherein in the first configuration the at least one limb is wrapped around at least a portion of the body and wherein in the second configuration the at least one limb extends outward from the body; and interacting with an adjacent tissue surface with an active portion of the ingestible device during and/or after deployment of the at least one limb.

It should be appreciated that the foregoing concepts, and additional concepts discussed below, may be arranged in any suitable combination, as the present disclosure is not limited in this respect. Further, other advantages and features of the present disclosure will become apparent from the following detailed description of various non-limiting embodiments when considered in conjunction with the accompanying figures.

### BRIEF DESCRIPTION OF DRAWINGS

Non-limiting embodiments of the present disclosure will be described by way of example with reference to the accompanying figures, which are schematic and are not intended to be drawn to scale. In the figures, each identical or nearly identical component illustrated is typically represented by a single numeral. For purposes of clarity, not every component is labeled in every figure, nor is every component of each embodiment of the disclosure shown where illustration is not necessary to allow those of ordinary skill in the art to understand the disclosure. In the figures:
Fig. 1A is a perspective view of an ingestible device in a first configuration according to one illustrative embodiment;
Fig. 1B is a perspective of the ingestible device of Fig. 1A in a second configuration;
Fig. 2A is a perspective view of an ingestible device in a first configuration according to one illustrative embodiment;
Fig. 2B is a perspective of the ingestible device of Fig. 2A in a second configuration;
Fig. 3 is a perspective view of an ingestible device transitioning from a first orientation to a second orientation according to one illustrative embodiment;
Fig. 4A is a cross sectional view of an ingestible device delivering a therapeutic to adjacent tissue according to one illustrative embodiment;
Fig. 4B is a cross sectional view of an ingestible device delivering a therapeutic to adjacent tissue according to the embodiment of Fig. 4A;
Fig. 5A is a perspective view of an ingestible device in a first configuration according to one illustrative embodiment;
Fig. 5B is a perspective of the ingestible device of Fig. 5A in a second configuration;
Fig. 5C is a side view of the ingestible device of Fig. 5A in a second configuration with a curved needle deployed into adjacent tissue;
Fig. 6A is a perspective view of an ingestible device in a first configuration according to one illustrative embodiment;
Fig. 6B is a perspective of the ingestible device of Fig. 6A in a second configuration; and
Fig. 7 is a is a flow chart illustrating a method of using an ingestible device according to one illustrative embodiment.

### DETAILED DESCRIPTION

In the fields of gastroenterology and oral drug delivery, an ingestible delivery device may be desirable to interact with (e.g., deliver a payload, bring a sensor into proximity with tissue, or otherwise bring an active portion of a device into proximity or contact with tissue) the walls of the target organ. For example, an ingestible delivery device may aid in overcoming barriers that may prevent diffusion and absorption of certain classes of therapeutic molecules, such as biologics or monoclonal antibodies. Also, the ingestible device may allow for the accurate collection of data related to GI tissue, since a sensor associated with the ingestible device may be able to directly interact with the tissue within the GI tract of the subject. As used herein, the GI tract may include the esophagus, the stomach, the duodenum, the jejunum, the small intestine, and the large intestine of a subject. Typically, a clinician may employ mechanical means, such as solid needles or liquid jets, to interact directly with the submucosal layer of gastrointestinal tissues, which may allow a device to circumvent such barriers. Such tissue penetration, however, may be rendered ineffective unless a mostly perpendicular orientation in relation to the tissue is achieved. In certain organs, such as the stomach, it may be challenging for such a perpendicular orientation to be achieved using ingestible devices since direct access to such tissue is typically provided via invasive surgery.

In view of the above, the inventors have recognized the advantages of an ingestible device capable of orienting itself in a desired orientation after being ingested. Additionally, the inventors have recognized the advantages of an ingestible device capable of orienting an active portion of the ingestible device such that it is in contact with and/or oriented towards a target tissue. Moreover, such an ingestible device may be capable of interacting with the target tissue predictably, reliably, and/or autonomously. While in some instances, the ingestible device may include features that allow the ingestible device to predictably be oriented in a single, stable configuration, optionally, in some instances, the ingestible device may be capable of predictably orienting in one of multiple stable orientations relative to the underlying supporting tissue. To provide this desired functionality, a device may include one or more limb(s) that may be deployed such that the one or more limbs extend outwards from the body to define one, two, or any other suitable number of desired stable orientations of the device relative to the underlying tissue depending on the application.

As noted above, in some embodiments, to orient an ingestible device within the GI tract of a subject, the ingestible device may include one or more deployable limbs. The limb(s) may be capable of deploying from a first configuration to a second configuration within the GI tract of a subject when exposed to a predetermined condition. For example, the ingestible device may initially be in a first configuration in which the limb(s) may be wrapped around a body of the ingestible device. When the limb(s) deploy into the second configuration, the limb(s) may extend outwardly away from the body. Thus, when the limb(s) are deployed, the limb(s) may serve to stabilize the ingestible device in a desired orientation relative to the underlying tissue. In some embodiments, the ingestible device may include an active portion disposed on an exterior of the body. The desired orientation may be one such that the active portion of the ingestible device is oriented towards and/or is in contact with the underlying adjacent tissue. In some embodiments, the limb(s) may be configured to deploy radially outwards from the first configuration such that the limb(s) are at least partially perpendicular to a longitudinal axis of the body when they are in the second extended configuration, though other orientations of the one or more deployable limbs are also contemplated.

As described herein, in some embodiments, the limb(s) may serve to stabilize the ingestible device in a desired orientation. For example, in some embodiments, the limb(s) may be configured such that the ingestible device may reach an equilibrium position within the GI tract of a subject under the influence of a gravitational force. The limb(s) may serve to stabilize the ingestible device in such an equilibrium position. Alternatively or in addition, the limb(s) may be configured to deploy in such a manner that the limb(s) may serve to move the ingestible device into a desired orientation regardless of the initial orientation of the ingestible device on a supporting tissue surface within the GI tract of the subject.

In some embodiments, it may be desirable for the limb(s) to deploy such that the limb(s) control the movement of the ingestible device relative to the adjacent tissue. For example, in some embodiments, an ingestible device may include two or more limbs configured to extend outward from a body of the ingestible device in a second (e.g., deployed) configuration. In such embodiments, a first limb of the two or more limbs may prevent the ingestible device from rotating is a first direction and a second limb of the two or more limbs may prevent the ingestible device from rotating in a second direction opposite the first direction. Thus, the two or more limbs may serve to stabilize the ingestible device in a desired orientation.

In some embodiments, it may be desirable to deploy limbs of an ingestible device in a predetermined manner and/or order which may aid in providing a desired orientation of the device relative to an underlying portion of tissue. For example, the limbs may be configured such that a first limb deploys before a second limb deploys. This sequential deployment of two or more limbs may be provided in a number of different ways. For example, in some embodiments, the limbs may overlap in the first configuration such that the first limb prevents the second limb from deploying until the first limb is deployed. However, other embodiments using different combinations of materials may be used. For instance, a limb made from an elastic material may deploy prior to a limb made from a shape memory material. Similarly, limbs made from materials that undergo different rates of expansion in response to an applied stimulus (e.g., heat, water absorption, etc.), may deploy out from a device at different times. Similarly, different thickness and/or types of adhesives and/or limb deployment triggers may be used to sequentially release the different limbs as the disclosure is not limited to any particular construction to provide this desired functionality.

Alternatively or in addition to the above, in some embodiments, the deployment of the limbs may be dependent on a timing of exposure of the limbs to a predetermined condition. In such embodiments, the arrangement of the limbs may serve to control the point at which each limb is exposed to the predetermined condition. For example, in some embodiments, a first limb may overlap (at least in part) the second limb such that the first limb prevents the second limb from being exposed to the predetermined condition. Particularly, in some embodiments, the limbs may be configured to deploy when wet (e.g., exposure to a liquid may cause the limbs to swell, differentially expand, or otherwise open). Accordingly, in such embodiments the overlapping placement of the first limb may prevent the second limb from deploying (e.g., by preventing the second limb from becoming wet) until the first limb is at least partially deployed such that the second limb is exposed to the surrounding environment. Alternatively or in addition, the deployment timing of the two limbs may be based at least in part on differences between the material properties of the first and second limbs. For example, the materials for each limb may be selected such that the material of the first limb deploys before and/or more quickly than the material of the second limb when both limbs are exposed to the same predetermined deployment condition.

It should be understood that the limb(s) of an ingestible device may be triggered to expand between a retracted and expanded configuration after exposure to a predetermined condition in the GI tract in any suitable manner. For example, the limb(s) may be formed from a shape memory material, a swellable material such as a swellable polymer that swells upon exposure to a liquid; and/or any other appropriate material capable of transitioning between a first retracted state and a second expanded state when exposed to a predetermined condition. Alternatively or in addition, the ingestible device may include a dissolvable outer coating and/or capsule capable of dissolving at a predetermined location within the GI tract after which the one or more limbs may deploy. In yet another embodiment, the one or more limbs may be locked in place by a limb deployment trigger such as a dissolvable limb deployment trigger or adhesive, an actively operated mechanical lock, and/or any other appropriate limb deployment trigger configured to release the one or more limbs after exposure to a predetermined condition within the GI tract.

In the various embodiments disclosed herein, the limb(s) may be formed from any suitable material or materials. For example, in some embodiments, the limb(s) may be formed from carbohydrates, hydrating starches, and/or lipids. In other embodiments, the limb(s) may be formed from cellulose, proteins, and/or gelatin (e.g., gelatin that may change in shape as a function of hydration). In further embodiments, the limb(s) may be formed from metals and/or polymers (e.g., shape memory materials and/or materials that differentially expand based on temperature). In yet further embodiments, the limb(s) may be formed from polymer bilayers such as polymer bilayers that may differentially swell when exposed to a predetermined condition and/or electroactive polymer bilayers. Of course, any suitable combination of the above or other appropriate materials may be employed, depending on the application as the disclosure is not so limited in this regard.

As discussed herein, exposure of an ingestible device to a predetermined condition may trigger deployment of one or more limbs and/or delivery of an active pharmaceutical ingredient (API). In some embodiments, the predetermined condition includes one or more of a predetermined time after ingestion of the ingestible device, a predetermined location in the GI tract, physical contact with the GI tract, physical manipulation in the GI tract (e.g., compression via peristalsis), one or more characteristics of the GI tract (e.g., pH, pressure, temperature, etc.), or combinations thereof. As noted above, in some embodiments, the predetermined condition may be associated with a predetermined location along the GI tract of a subject (e.g., esophagus, stomach, small intestine, large intestine, etc.). Moreover, in some embodiments, the predetermined condition may include a threshold pH value. Particularly, the limb(s) may be configured such that they deploy when exposed to a pH above and/or below the threshold pH value. Alternatively or in addition, the predetermined condition may include exposure to an aqueous environment. Particularly, the limb(s) may deploy when they become wet. In some embodiments, the predetermined condition may include exposure to enzymes. Particularly, the limb(s) may be configured to deploy when exposed to one or more particular enzymes present within a desired location of a subject's GI tract. In other embodiments, the limb(s) may be configured to deploy when exposed to a temperature greater than, less than, and/or equal to a threshold temperature. In some embodiments, combinations of the predetermined conditions described above may be employed, depending on the application. For example, in some embodiments, a first predetermined condition may trigger the deployment of limbs as described herein and a second predetermined condition may trigger the delivery of an API as described herein. In various embodiments, the first predetermined condition may be the same as or different from the second predetermined condition. Further, the time and/or location within the GI tract at which the first and second predetermined conditions occur may each be the same or different. In some embodiments, a single predetermined condition may trigger both the deployment of limbs and the delivery of API. Of course, any suitable predetermined condition or conditions may be employed with any of the embodiments described herein as the disclosure is not so limiting in this regard. Specific types of triggers are discussed further below.

In some embodiments, the limb(s) may have an overall length, such as a maximum dimension along a longitudinal axis of the limb(s). The overall length may be greater than or equal to 2 mm, 4 mm, 6 mm, and/or another appropriate length. Correspondingly, the limb(s) may have an overall length less than or equal to 16 mm, 18 mm, 20 mm, and/or another appropriate length. Combinations of the above-noted ranges are contemplated, including, but not limited to, overall lengths between 2 mm and 20 mm, between 4 mm and 18 mm, and between 6 mm and 16 mm. In some embodiments, the overall length of the limb(s) is 10 mm. Of course, any suitable overall length or combination of lengths may be employed, depending on the application.

In some embodiments, the limbs of an ingestible device may have the same overall length though this need not be the case. In some embodiments, the length of the limbs may be different. Of course, any suitable combination of overall limb lengths may be employed, depending on the application.

In some applications, a length of the one or more limb(s) of a device that is configured to roll across a supporting surface during deployment may determine how far the limb(s) may move the ingestible device within the GI tract of the subject (e.g., as described herein) during deployment. For example, longer limb(s) may serve to move the ingestible device a greater distance, while shorter limb(s) may serve to move the ingestible device a shorter distance. Accordingly, any suitable limb length may be employed, depending on the application.

In some embodiments, the limb(s) may have an overall width, such as a maximum dimension parallel to a lateral axis of the limb(s) which may be perpendicular to the longitudinal axis of the limb in some embodiments. The overall width may be greater than or equal to 1 mm, 2 mm, 3 mm, and/or another appropriate width. Correspondingly, the limb(s) may have an overall width less than or equal to 8 mm, 9 mm, 10 mm, and/or another appropriate width. Combinations of the above-noted ranges are contemplated, including, but not limited to, overall widths between 1 mm and 10 mm, between 2 mm and 9 mm, and between 3 mm and 8 mm. In some embodiments, the overall width of the limb(s) is 5 mm. Of course, any suitable overall width or combination of widths may be employed, depending on the application.

In some embodiments, the limb(s) may have an overall thickness measured in a direction that is perpendicular to both the longitudinal and lateral axes of the limb in the deployed configuration in some embodiments. The overall thickness may be greater than or equal to 1 mm, 1.2 mm, 1.3 mm, and/or another appropriate thickness. Correspondingly, the limb(s) may have an overall thickness less than or equal to 1.8 mm, 1.9 mm, 2 mm, and/or another appropriate thickness. Combinations of the above-noted ranges are contemplated, including, but not limited to, overall thicknesses between 1 mm and 2 mm, between 1.2 mm and 1.9 mm, and between 1.3 mm and 1.8 mm. In some embodiments, the overall thickness of the limb(s) is 1.5 mm. Of course, any suitable overall thickness or combination of thicknesses may be employed, depending on the application.

The limb(s) may be attached to a body of an ingestible device in any suitable manner. For example, in some embodiments, the limb(s) may be separately formed from and are attached to the body via mechanical clamping, adhesive attachment, ultrasonic welding, fasteners, and/or any other appropriate type of connection. In other embodiments, the limb(s) may be integrally formed with the body. Of course, any combination of the above constructions may be employed depending on the application.

In some embodiments, the limb(s) may be configured to detach from a body of an ingestible device after the limb(s) are deployed (e.g., as described herein). Particularly, the limb(s) may detach at a predetermined time (e.g., after ingestion and/or deployment of the limbs) and/or after a predetermined condition is achieved (e.g., within a GI tract of a subject). In some embodiments, the attachment of the limb(s) may be configured to degrade after a certain time (e.g., after ingestion and/or after deployment of the limbs) and/or once a predetermined condition is achieved (e.g., within a GI tract of a subject). Thus, the limb(s) may be configured to detach from the body.

An ingestible device according to the present disclosure may include an active portion configured to interact with adjacent tissue, which may be in contact with the active portion in some embodiments, in any suitable manner. For example, in some embodiments, the ingestible device may include an active portion configured to electrically stimulate adjacent tissue. Particularly, in some embodiments, a clinician may wish to influence the metabolism of a subject, by directing the body of the subject to release certain hormones. For example, in some instances, a clinician may wish to influence the levels of the hormone Ghrelin within a subject to curb the appetite of the subject. As will be appreciated by one of skill in the art, electrical stimulation of the stomach mucosa may have a significant effect on the levels of the hormone Ghrelin present in a subject. Thus, it may be desirable for an ingestible device to provide electrical stimulation to tissue within the GI tract of a subject. Accordingly, the active portion of an ingestible device may include one or more electrodes disposed on an exterior surface of the ingestible device configured to electrically stimulate adjacent tissue (e.g., when in contact with the adjacent tissue).

In other embodiments, the ingestible device may include an active portion configured to sense one or more parameters of adjacent tissue. Particularly, in some embodiments, the active portion of the ingestible device may include contact based and/or non-contact sensors configured to sense one or more parameters of the adjacent tissue. For example, the sensors may be adapted to measure tissue resistivity, the concentration of one or more biomarkers, pH and/or any other suitable parameter or parameters. Specific non-limiting examples of sensors that may be used may include, but are not limited to, electrodes, biochemical sensors, pH sensors and/or any other suitable type of sensor. It should be appreciated that any suitable type of sensor may be employed, and any suitable parameter may be measured by the sensor, as the disclosure is not so limited in this regard.

In further embodiments, the ingestible device may include an active portion configured to deliver a drug to adjacent tissue. The ingestible device may be configured to deliver an active pharmaceutical ingredient via one or more of a coating on an exterior portion of the device, a jet injector, a needle injector, a solid dosage form configured for penetrating tissue, a microneedle patch, or any other suitable delivery mechanism. Specific examples of potential active pharmaceutical ingredients (APIs) are detailed further below. Additionally, in some embodiments, an alternative payload may be deployed to the target location including payloads including, but not limited to, a sensor, a virus, a bacterium, and/or any other suitable payload as the present disclosure is not so limited in this regard.

In some embodiments, an active portion may include one or more potential energy sources configured to pressurize and expel an API through or from the device. For example, an active portion may include one or more potential energy sources for dispensing and/or delivering an API. A potential energy source may be configured to store energy in the ingestible device using physical, chemical, electrochemical, and/or any other appropriate type of energy storage. In some embodiments, the potential energy source may be associated with a reservoir such that operation of the potential energy source pressurizes the API within the reservoir to cause the API to flow out of the ingestible device through one or more jet outlets, one or more needles, and/or any other appropriate delivery mechanism. For example, in some embodiments, a potential energy source may be operatively coupled with a moveable piston, membrane, and/or other structure(s) that is configured to pressurizing a volume of fluid or API in order to drive the API from the device (e.g., from a reservoir within the device) through a jet outlet, a needle, and/or another delivery mechanism. Of course, while several examples of delivery mechanisms are described above, it should be understood that any construction capable of delivering an API from an ingestible device to a target location and/or tissue may be used with the currently disclosed ingestible devices as the disclosure is not so limited.

In some embodiments, the potential energy source may include a compressed gas. The compressed gas may be directly stored in the ingestible device, or the compressed gas may be generated via a chemical reaction or phase change. For example, in some embodiments dry ice may be stored in a chamber of the ingestible device so that compressed gas is generated as the dry ice sublimates. Alternatively, a compressed gas may be provided to a desired chamber prior to sealing an ingestible device. In some embodiments, the potential energy source may be a spring (e.g., a compressed compression spring). In some embodiments, the potential energy source may include a reaction chamber. For example, the reaction chamber may allow an acid and base to be combined to generate gas, leading to the expulsion of API. In some embodiments, a detonation catalyst may cause detonation of an explosive material located within a chamber to generate pressurized gas for expelling the API from the ingestible device. Of course, any suitable reaction or other potential energy source may be employed to pressurize and drive an API out from an ingestible device as the present disclosure is not so limited.

In view of the above, in some embodiments, an active portion of an ingestible device may be configured to deliver a drug dose directly to tissue within the GI tract of a subject once the device is appropriately oriented within the GI tract. Particularly, in some embodiments, a clinician may employ an ingestible device containing one or more extendable needles, microneedles (and/or microneedle patches), and/or needle-free jet injectors configured to deliver a predetermined dosage of an active pharmaceutical ingredient (API) to the tissue of the GI tract of a subject. In such an embodiment, it may be preferable for the ingestible device to deliver a dose of the API when an associated portion of the device, e.g., an outlet or other active portion of the device, is oriented towards and/or in contact with the tissue the device is supported on relative to a direction of gravity. In some instances, the API may be delivered via an outlet or needle that is at least partially oriented in a direction parallel the direction of gravity when the device is oriented using the self-orienting features described herein which may help to deliver the API dose into the desired tissue within the GI tract.

As will be appreciated by one of skill in the art, an active portion of an ingestible device may perform any suitable function. For example, in some embodiments, an active portion of an ingestible device may include biopsy tools configured to collect a sample of a tissue adjacent to the ingestible device within the GI tract of a subject. Of course, the active portion of the ingestible device may perform any appropriate function, depending on the application, as the disclosure is not so limited in this regard.

The active portion of the ingestible device may be positioned on an ingestible device in any appropriate position and/or orientation such that the active portion may be oriented towards and/or placed into contact with adjacent tissue of the GI tract of the subject during operation. For example, in some embodiments, the active portion is positioned on a portion of a body of the device that is oriented towards and/or in contact with an adjacent tissue surface supporting the device once the one or more limbs had been deployed. In other embodiments, an active portion of the ingestible device may be located on the one or more limb(s) of the device (e.g., the limb(s) may include one or more sensors, coatings, microneedle patches, needles, or any other appropriate type of active portion as disclosed herein). In some embodiments, the delivery device may include several active portions including active portions on the body and/or the limb(s). Of course, the active portion or portions may be configured in any suitable manner, depending on the application. As used herein, the term "active pharmaceutical ingredient" (also referred to as a "drug" or "therapeutic agent") refers to an agent that is administered to a subject to treat a disease, disorder, or other clinically recognized condition, or for prophylactic purposes, and has a clinically significant effect on the body of the subject to treat, prevent, and/or diagnose the disease, disorder, or condition. The active pharmaceutical ingredient may be delivered to a subject in a quantity greater than a trace amount to affect a therapeutic response in the subject. In some embodiments, active pharmaceutical ingredients (API's) can include, but are not limited to, any synthetic or naturally occurring biologically active compound or composition of matter which, when administered to a subject (e.g., a human or nonhuman animal), induces a desired pharmacologic, immunogenic, and/or physiologic effect by local and/or systemic action. For example, useful or potentially useful within the context of certain embodiments are compounds or chemicals traditionally regarded as drugs, vaccines, and biopharmaceuticals. Certain such APIs may include molecules such as proteins, peptides, hormones, nucleic acids, gene constructs, etc., for use in therapeutic, diagnostic, and/or enhancement areas. In certain embodiments, the API is a small molecule and/or a large molecule. Further, while according to exemplary embodiments described herein an ingestible device may generate an incompressible jet of a liquid API, in other embodiments a jet of an API generated by an ingestible device may be formed of gases, viscous fluids, aerosolized powders, and/or other appropriate type of API, as the present disclosure is not so limited. Accordingly, it should be understood that the API's described herein are not limited to any particular type of API.

According to exemplary embodiments described herein, a limb deployment trigger of an ingestible device may be configured to deploy the limb(s) of the device in the GI tract of a subject under a predetermined condition as described herein. In some embodiments, the limb deployment trigger may be a passive component configured to interact with the environment of the GI tract to actuate the ingestible device. For example, in some embodiments the limb deployment trigger may be a dissolvable material, configured to dissolve in the GI tract. The dissolvable material may surround the ingestible device, cover at least a portion of a limb, and/or be used to adhere a portion of a limb to a body of the device prior to ingestion and may have a certain thickness and/or shape that at least partly determines the rate at which the material dissolves and ultimately actuates the limb(s) of the device. Appropriate materials for a dissolvable limb deployment trigger may include, but are not limited to, sugar alcohols such as disaccharides (e.g. Isomalt), water soluble polymers such as Poly-vinyl alcohol, enteric coatings, time-dependent coatings, enteric and time-dependent coatings, temperaturedependent coatings, light-dependent coatings, and/or any other appropriate material capable of being dissolved within the GI tract of a subject. In some embodiments, a limb deployment trigger may include a triggerable membrane including EDTA, glutathione, or another suitable chemical. In some embodiments, a sugar alcohol trigger may be employed in combination with an enteric coating configured to protect the sugar alcohol trigger until the ingestible device is received in the GI tract of a subject. In some embodiments, the limb deployment trigger may include a pH responsive coating to assist with delaying triggering until after ingestion. In some embodiments, the limb deployment trigger may be a sensor that detects one or more characteristics of the GI tract. For example, a sensor detecting contact with a GI mucosal lining may be used to deploy the limb(s) of the device. In embodiments where a sensor is employed, the limb deployment trigger may also include an active component that moves in response to a predetermined condition being detected by the sensor. Of course, any suitable active or passive limb deployment trigger may be employed for an ingestible device, as the present disclosure is not so limited.

Additionally or alternatively, an API delivery trigger of an ingestible device may be configured to actuate an active portion of an ingestible device to deliver an API from the ingestible device to the GI tract of a subject under a predetermined condition as described herein. In some embodiments, the API delivery trigger may include a passive component configured to interact with the environment of the GI tract to actuate the ingestible device. For example, in some embodiments the API delivery trigger may include a sugar plug, or other dissolvable material, configured to dissolve in the GI tract. The dissolvable plug may have a certain thickness and/or shape that at least partly determines the speed at which the sugar plug dissolves and ultimately actuates the ingestible device. In some embodiments, the API delivery trigger may have an oval shape, an egg shape, a spherical shape, an elliptical shape, a cylindrical shape, a conical shape, or a spherocylindrical shape. In another embodiment, the API delivery trigger may be at least partially formed by an enteric coating. For example, in some embodiments, an API delivery trigger may include both a sugar plug and an enteric coating, as the present disclosure is not so limited. Other appropriate materials for a dissolvable API delivery trigger may include, but are not limited to, sugar alcohols, such as disaccharides (e.g. Isomalt), water soluble polymers, such as Poly-vinyl alcohol, enteric coatings, time-dependent coatings, enteric and time-dependent coatings, temperaturedependent coatings, light-dependent coatings, and/or any other appropriate material capable of being dissolved within the GI tract of a subject. In some embodiments, an API delivery trigger may include a triggerable membrane including EDTA, glutathione, or another suitable chemical. In some embodiments, a sugar alcohol trigger may be employed in combination with an enteric coating configured to protect the sugar alcohol trigger until the ingestible device is received in the GI tract of a subject. In some embodiments, the API delivery trigger may include a pH responsive coating to assist with delaying triggering until after ingestion. In some embodiments, the API delivery trigger may be a sensor that detects one or more characteristics of the GI tract. For example, a sensor detecting contact with a GI mucosal lining may be used to actuate the device. Other types of API delivery triggers may include an electrical timer, a light sensor, an enzymatic sensor, a conductivity sensor, a pH sensor, a pressure sensor, a temperature sensor, and/or any other appropriate sensor or construction capable of providing a signal to a processor or closing an electrical circuit associated with a processor or other portion of the device when that the device is exposed to one or more predetermined conditions corresponding to a desired target location within the GI tract or other anatomical structure of a subject. Accordingly, it should be understood that the API delivery triggers disclosed herein are not limited to any specific type or construction of trigger.

In some embodiments, an ingestible device is sized and shaped to be ingested by a subject. Accordingly, the ingestible device may be appropriately small so that the ingestible device may be easily swallowed and subsequently pass through the GI tract including the esophagus and pyloric opening within the stomach. In some embodiments, an ingestible device prior to expansion of the one or more limbs may over an overall length, such as a maximum dimension along a longitudinal axis of the device, less than or equal to 40 mm, 30 mm, 20 mm, 10 mm, 5 mm, and/or another appropriate length. Correspondingly, an ingestible device may have an overall length greater than or equal to 3 mm, 5 mm, 10 mm, 20 mm, 25 mm, and/or another appropriate length. Combinations of the above-noted ranges are contemplated, including, but not limited to, overall lengths between 5 mm and 30 mm, 10 mm and 30 mm, 5 mm and 20 mm, as well as 5 mm and 10 mm. In some embodiments, an ingestible device prior to expansion of the one or more limbs may have a maximum external transverse dimension, such as a diameter, less than or equal to 11 mm, 10 mm, 7 mm, 5 mm, and/or another appropriate diameter. Correspondingly, an ingestible device prior to expansion of the one or more limbs may have a maximum external transverse dimension greater than or equal to 3 mm, 5 mm, 7 mm, 9 mm, and/or another appropriate diameter. Combinations of the above-noted ranges are contemplated, including, but not limited to, maximum external transverse dimensions between 5 mm and 11 mm, 5 and 7 mm, as well as 7 mm and 11 mm. In some embodiments, an ingestible device prior to expansion of the one or more limbs may have an overall volume less than or equal to 3500 mm³, 3000 mm³, 2500 mm³, 2000 mm³, 1500 mm³, 1000 mm³, 750 mm³, 500 mm³, 250 mm³, 100 mm³, and/or any other appropriate volume. Corresponding, an ingestible device may have an overall volume greater than or equal to 50 mm³, 100 mm³, 250 mm³, 500 mm³, 750 mm³, 1000 mm³, 1500 mm³, 2000 mm³, 2500 mm³, and/or any other appropriate volume. Combinations of the above-noted ranged are contemplated, including, but not limited, volumes between 1000 mm³ and 3000 mm³, 1500 mm³ and 3000 mm³, 50 mm³ and 500 mm³, 50 mm³ and 100 mm³, as well as 2000 mm³ and 3000 mm³. Of course, any suitable overall length, maximum external transverse dimension, and volume may for an ingestible delivery device may be employed, as the present disclosure is not so limited.

According to exemplary embodiments described herein, an ingestible device may be configured to be submerged into a liquid so as to enable the ingestible device to sink to a bottom of a body lumen (e.g., subsequent to ingestion). In accordance herewith, in some embodiments, the ingestible device may have a density larger than 1 g/cm³, 2 g/cm³, 3g/cm³, 5 g/cm³, 7 g/cm³, and/or another appropriate density.

According to exemplary embodiments described herein, the ingestible device is administered to a subject orally. In other embodiments, the ingestible device may be administered, rectally, vaginally, nasally, or in any other appropriate fashion as the present disclosure is not so limited.

Turning to the figures, specific non-limiting embodiments are described in further detail. It should be understood that the various systems, components, features, and methods described relative to these embodiments may be used either individually and/or in any desired combination as the disclosure is not limited to only the specific embodiments described herein.

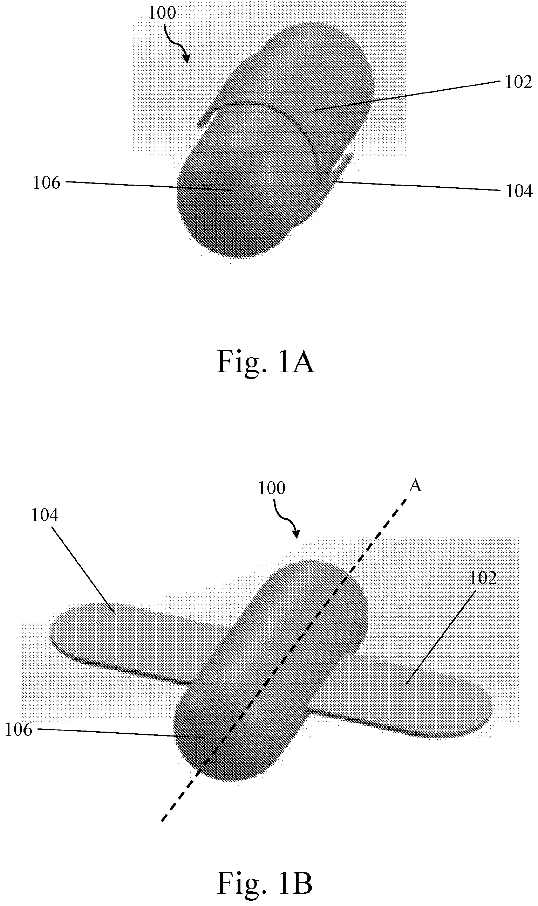

Figs. 1A-1B show perspective views of an ingestible device 100 in a first, undeployed configuration and a second, deployed configuration respectively. The ingestible device 100 includes first and second limbs 102, 104 attached to an elongate body 106 of the ingestible device 100. In an initial configuration, for example as shown in Fig. 1A, the first and second limbs 102, 104 may be wrapped around at least a portion of the elongate body 106. Accordingly, a profile and/or radius of curvature of a limb in an initial configuration may correspond to a profile or radius of curvature of the elongate body. In some embodiment, this may correspond to a profile or radius of curvature of the elongate body in a direction that is at least partially perpendicular to a longitudinal axis of the elongate body. Each of the first and second limbs 102, 104 may be configured to change its respective profile and/or radius of curvature during deployment from the initial configuration to the deployed configuration. For example, in some embodiments, a radius of curvature of a limb may increase and/or a profile of a limb may change during deployment until the limb has achieved a deployed configuration. This deployment may include a transition where the limbs unwrap from around and rotate radially outwards from the elongated body. In some embodiments, this may correspond to at least a portion of the limb rotating about an axis that is at least partially parallel to a longitudinal axis of the elongate body. After deployment, in some embodiments, a limb may have a desired profile or radius of curvature in the deployed configuration. For example, in the embodiment shown in Figs. 1A-1B, each of the first and second limbs 102, 104 may have a substantially flat profile (i.e., a substantially infinite radius of curvature) in the deployed configuration. It will be appreciated that although flat profiles are shown in the figures, a deployed configuration or a desired profile thereof may include any appropriate shape and/or curvature.

In the depicted embodiment, the limbs are wrapped partially around the elongated body in a direction that is perpendicular to the longitudinal axis of the device, though other orientations may also be used. In the depicted embodiment, the limbs are aligned with one another along a longitudinal length of the device and extend out from the device in the deployed configuration in directions that are at least partially oriented away from one another, and in some embodiments extend outwards from the body in opposite directions. However, embodiments in which the limbs are not aligned with one another are also contemplated as elaborated on further below. The first and second limbs 102, 104 may be configured to by deployable such that when first and second limbs 102, 104 are deployed, the first and second limbs 102, 104 extend away from the elongate body 106. In some embodiments, the first and second limbs 102, 104 may deploy such that they extend radially outwards from the elongated body and are approximately perpendicular to a longitudinal axis A of the body 106, for example as shown in FIG. 1B. However, other deployment directions are also contemplated.

In some embodiments, the ingestible device 100 may include features to control the deployment timing of the first and second limbs 102, 104. For example, in some embodiments, the first and second limbs 102, 104 may be longer than those shown in Fig. 1A such that the first and second limbs 102, 104 are located on the elongate body 106 in a stacked configuration such that at least a portion of the first limb 102 is disposed on a portion of the second limb 104 in the first retracted configuration (e.g., when the first and second limbs 102, 104 are wrapped around the body 106 of the ingestible device 100). Thus, the first limb 102 may prevent the second limb 104 from deploying until the first limb 102 is at least partially deployed. Accordingly, the stacked configuration may serve to control the deployment timing of the first and second limbs 102, 104. Of course, the deployment timing of the first and second limbs 102, 104 may be controlled in any suitable manner, including those described previously above.

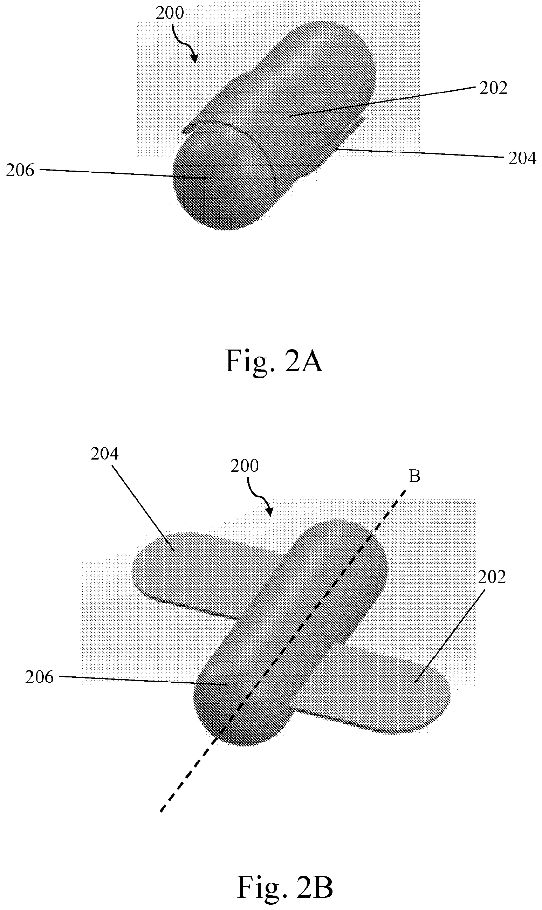

As noted above, in some embodiments, an ingestible device may include offset limbs. For example, as illustrated in Figs. 2A-2B, an ingestible device 200 may include first and second limbs 202, 204 in a configuration wherein the first limb 202 and the second limb 204 do not overlap with one another. Specifically, as illustrated, in some embodiments, the first limb 202 may be offset from the second limb 204 in a direction parallel to a longitudinal axis B of the ingestible device. Additionally, similar to the above embodiment, the limbs in the extended configuration may be oriented in directions that are at least partially oriented away from one another, and in some instances are oriented in opposite directions. Further, in some such embodiments, the first and second limbs 202, 204 may be configured to deploy simultaneously. Of course this need not be the case, as the first and second limbs 202, 204 may be deployed with any suitable timing (e.g., by employing the methods described herein), depending on the application.

In the embodiments illustrated in Figs. 1A-2B, ingestible devices are shown having two limbs. However, an ingestible device may include one limb, three limbs, or four or more limbs, depending on the application.

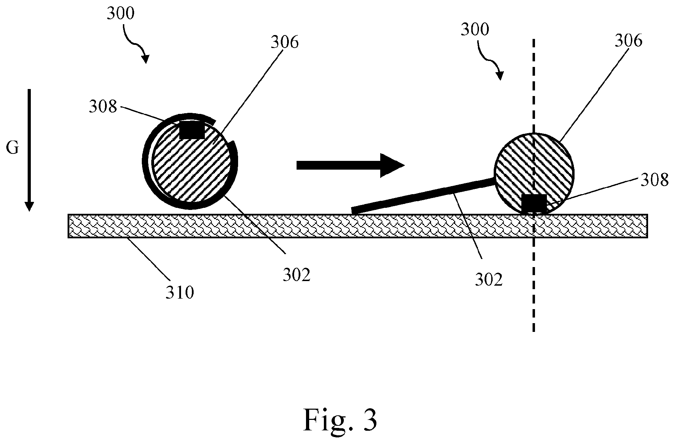

In some embodiments, an ingestible device may include an active portion. For example, as illustrated in Fig. 3, an ingestible device 300 may include an active portion 308. The active portion 308 may be configured to perform a desired function on an adjacent tissue 310. For example, the active portion 308 may correspond to: a sensor that is configured to sense one or more parameters of the adjacent tissue 310; a jet outlet, needle, solid microneedles comprising an API, an API coating on an exterior portion of the device, and/or any other appropriate configuration capable of delivering a dose of an API directly to tissue 310; one or more electrodes configured to electrically stimulate the adjacent tissue 310; or any other appropriate device construction configured to perform one or more desired functions on the tissue it is oriented towards and/or in contact with (e.g., including but not limited to the other functions described herein).

In some embodiments, the function performed by the active portion 308 may be facilitated if the active portion 308 is proximate to or in contact with the adjacent tissue 310. Accordingly, the ingestible device 300 may include features that allow the ingestible device 300 to be oriented such that the active portion 308 is in contact with or proximate to the adjacent tissue 310 when the device has been deployed. Particularly, in the embodiment illustrated in Fig. 3, the ingestible device 300 includes a deployable limb 302 configured to control the orientation of an elongated body 306, and correspondingly the active portion 308 of the ingestible device 300. In the depicted embodiment, the elongated body has a round cross section perpendicular to a longitudinal axis of the body, though other shapes may also be used. As described herein, it may be desirable for the active portion 308 to be oriented towards and/or placed into contact with the adjacent tissue 310 the device is disposed on. In the illustrated embodiment, the deployable limb 302 is configured to reorient the elongate body 306 by rolling the elongate body 306 along the surface of the adjacent tissue 310 (e.g., from the left to the right as illustrated) as the limb 302 is deployed. Specifically, the one or more limbs 302 may be attached to the body 306 at a suitable location; have a correspondingly selected length; and may be constructed to unfurl in a predetermined direction such that the active portion is oriented towards and/or is in contact with the adjacent tissue 310 when the limb 302 is deployed. For example, as the limb 302 is deployed (e.g., by unfurling), the limb 302 may press on the adjacent tissue 310. In turn, the adjacent tissue 310 may apply a torque (e.g., based at least in part on the length of the limb 302) on the body 306 of the ingestible device 300. The torque may then serve to move (e.g., by rotating) the elongate body such that it rolls across the surface until the active portion is oriented towards and/or in contact with the adjacent tissue 310. Alternatively or in addition, the active portion 308 may deposit a payload on the adjacent tissue 310 as the active portion 308 rolls across the tissue. For example, a patch or microneedles, a coating, or different sensors may come into contact with the adjacent tissue as the one or more active portions of the device are rolled across the surface of the tissue.

In addition to the above, the deployable limb 302 may serve to stabilize the elongate body 306 in the deployed orientation relative to the adjacent tissue 310. For example, the limb 302 may shift the center of gravity of the ingestible device such that the ingestible device achieves stable equilibrium under the influence of a gravitational force G. Accordingly, the active portion 308 may be maintained in a stable orientation towards and/or in contact with the adjacent tissue and/or in contact with the adjacent tissue 310. In some embodiments, the limb 302 may be configured to provide one or more stable configurations for the ingestible device 300. For example, in some embodiments, the limb 302 may provide a single stable configuration (e.g., to orient the active portion 308 towards and/or in contact with the adjacent tissue 310). To achieve a single stable orientation, the length and/or placement of the limb 302 may be set such that when the limb 302 is deployed, the body 306 is moved to a single predetermined orientation. However, in some embodiments, the limb 302 may be configured to provide bi-stable, tri-stable, and/or other multi-stable configurations, as the disclosure is not so limited in this regard.

In some embodiments, the limb 302 and the body 306 may be configured such that when the limb 302 is deployed, the limb 302 is offset from the active portion 308 by an offset angle. The offset angle α may be greater than or equal to 30 degrees, 40 degrees, 50 degrees, and/or another appropriate angle. Correspondingly, the offset angle may be less than or equal to 130 degrees, 140 degrees, 150 degrees, and/or another appropriate angle. Combinations of the above-noted ranges are contemplated, including, but not limited to, offset angles between 30 degrees and 150 degrees, between 40 degrees and 140 degrees, and between 50 degrees and 130 degrees. In some embodiments, for example the embodiment illustrated in Fig. 3, the angle is 90 degrees. Of course, any suitable overall offset angle or combination of offset angles may be employed, depending on the application.

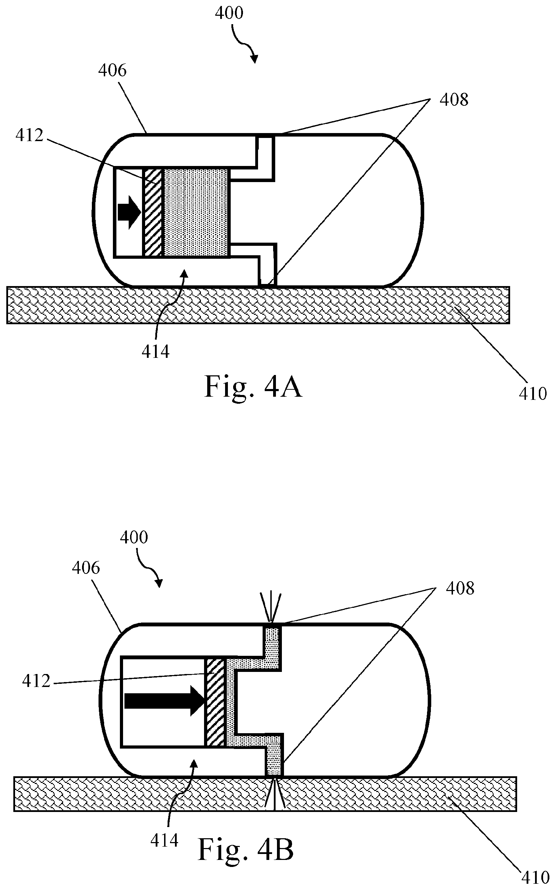

Turning to Figs. 4A-4B, in some embodiments, an ingestible device 400 may include multiple active portions 408 disposed on different portions of the device to increase a chance of at least one of the active portions being oriented towards, and in some instances in contact with, adjacent tissue underlying the device. In the illustrated embodiment, ingestible device 400 is adapted to deliver an API payload to tissue (e.g., within a GI tract) of a subject. Particularly, ingestible device 400 include two active portions in the form of two outlets 408. The API may be contained within a reservoir 414. In turn, the API may be configured to be deployed out of the outlets when a piston 412 compresses the reservoir. The piston may be actuated using a pressurized volume, a spring, a reaction, and/or using any other appropriate energy source. In either case, when the piston 412 is actuated, the API may be pressurized and expelled through the outlets 408, as illustrated in Fig. 4B. In the depicted embodiment, the API is deployed out of an inlet oriented towards the tissue and an outlet that is oriented into the interior space of the GI tract opposite from the tissue. However, as noted previously, any appropriate number of outlets or other active portions of the device may be used in any desired configuration as the disclosure is not so limited. Additionally, while in some instances, outlets 408 may be outlets configured to deploy a jet of the API, other possible configurations are also contemplated, including the use of needles, microneedles, coatings, electrodes, and/or any other suitable delivery mechanism for an API as described previously above.

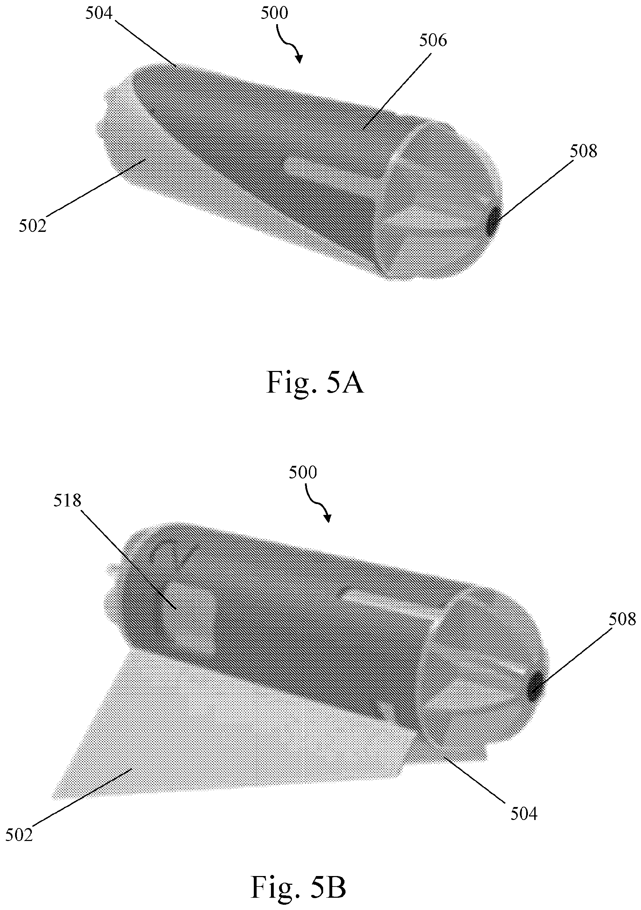

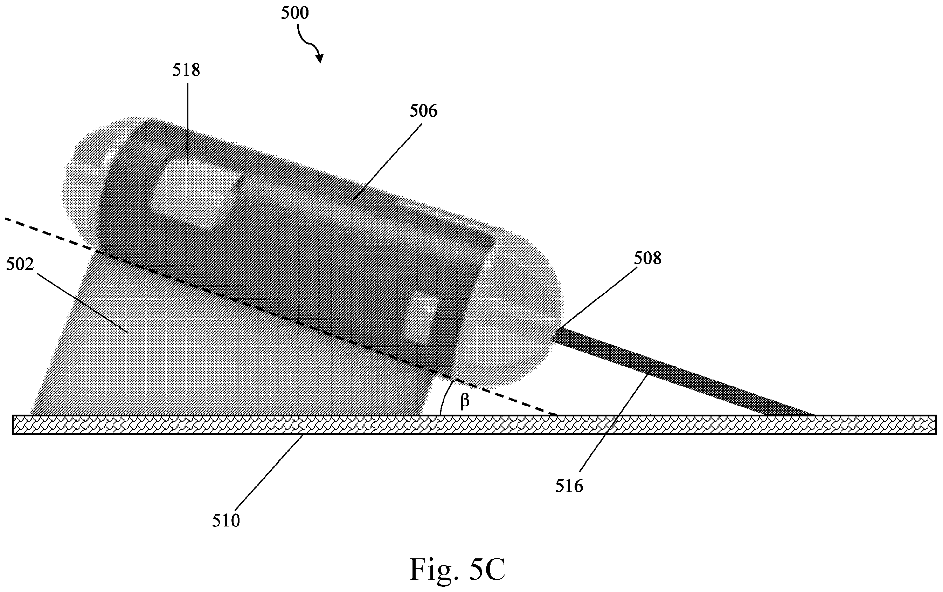

As will be appreciated by one of skill in the art, limbs may be oriented on an ingestible device in any suitable manner. For example, as illustrated in Figs. 5A-5C, an ingestible device 500 may be configured with first and second limbs 502, 504 configured to angle an outlet 508 (e.g., an active portion) generally towards an adjacent tissue (e.g., tissue 510 as shown in Fig. 5C). The first and second limbs 502, 504 may begin in a first undeployed configuration, as shown in FIG. 5A. In the undeployed configuration, the first and second limbs 502, 504 may be folded around at least a portion of the body 506. When exposed to a predetermined condition (e.g., as described herein), the first and/or second limbs 502, 504 may deploy into the second configuration, as shown in Fig. 5B. In the second configuration, the first and/or second limbs 502, 504 may be formed in a generally trapezoidal shape having a short side disposed proximally to the outlet 508 and a long side disposed distally away from the outlet 508 such that a width of each limb extends along a majority of a length of the body of the device. The difference in length between the short side and the long side of each limb may serve to angle the body 506 relative to the adjacent tissue 510 at an angle β, such that the outlet 508, or other active portion of the device, is angled towards the adjacent tissue when the limbs are in the extended configuration.

In some embodiments and as shown, the deployment of the first and second limbs 502, 504 may expose an opening 518 formed in the body 506. The opening 518, or other portion of the body exposed due to deployment of the one or more limbs, may include an API delivery trigger configured to actuate an active portion of the device under a predetermined condition. As noted above, the predetermined condition which causes the API delivery trigger to actuate the active portion may be the same as or different from the predetermined condition which causes the limbs to deploy. In some embodiments, the API delivery trigger may correspond to a sugar plug and/or an enteric coating associated with the opening that is configured to dissolve in the GI tract. In the embodiment shown, dissolution of the API delivery trigger may actuate a deployable needle 516 and subsequent delivery of the API. In some embodiments, the API may be delivered from a reservoir within the body 506 using a potential energy source (as described herein), a moveable piston, a membrane, and/or any other configuration or delivery mechanism capable of delivering the API. For example, the API may be driven, by a moveable piston or a membrane, from a reservoir within the body 506 through the deployable needle 516 and into to the tissue 510.

In some embodiments and as shown in Fig. 5C, the deployable needle 516 may be configured to penetrate an adjacent tissue 510 prior to a payload (e.g., an API) being delivered to the adjacent tissue 510. Once the first and second limbs 502, 504 are deployed and the body 506 is oriented such that the outlet 508 is oriented towards the adjacent tissue 510 (e.g., as described herein), the deployable needle 516 may be extended out from the outlet 508 (e.g., an active portion) of the ingestible device 500 and pierce the adjacent tissue 510. The ingestible device 500 may then deliver the payload to the adjacent tissue 510 through the needle 516. Alternatively, the needle may include a portion of the needle that is made from an API, or includes an API disposed therein, such that piercing the tissue may deliver the API to the tissue. The deployable needle 516 may be formed in any appropriate shape or geometry. For example, in the embodiment shown, the deployable needle 516 may be a straight needle. In other embodiments, the deployable needle may be curved. In some such embodiments, an elastically curved deployable needle may be in a straightened, internally stressed state while contained within the body 506. In some such embodiments, deployment of the needle from the outlet 508 results in the needle returning to the curved unstressed state such that the needle curves from the outlet and into the adjacent underlying tissue. Alternatively, other appropriate materials such as shape memory materials may be used to provide desired shapes of a deployed needle or other structure.

In addition to the above, a needle may be deployed from an ingestible device in any appropriate fashion. For example, in some embodiment, the deployable needle 516 may be releasably retained within the ingestible device prior to triggering. For example, the needle may be retained in the body by a triggerable lock or other component while a spring or other component biases the needle towards a deployed configuration in which the needle 516 is extended out of the body 506. Activation of the API delivery trigger (e.g., dissolution of a sugar plug or enteric coating) may release the needle, thereby actuating the deployable needle 516. Of course, it will be appreciated that other mechanisms for releasably retaining the needle within the body may also be used, as the disclosure is not limited in this regard.

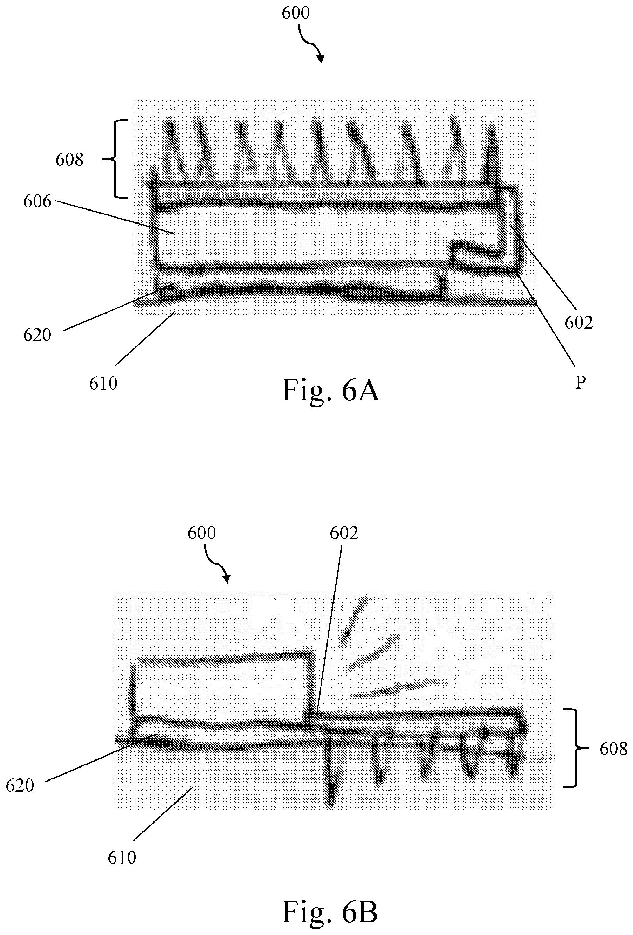

In some embodiments, an ingestible device 600 may include a body 606, a microneedle patch 608, and a limb 602 that connects the microneedle patch 608 to the body 606, as shown in Figs. 6A-6B. The limb 602 may be deployable such that when the limb is deployed, the microneedle patch may come into contact with an adjacent tissue 610 (e.g., to dispense an API or other suitable payload). In the initial undeployed configuration shown in Fig. 6A, the microneedle patch 608 is disposed above the body 606 with the microneedles pointed away from the adjacent tissue. In turn, the limb 602 is configured to extend about a point P, thus rotating the microneedle patch 608 about the point P, or other attachment point to the body, such that the microneedle patch 608 is oriented towards the adjacent tissue 610. Thus, during deployment of the limb 602, the microneedle patch 608 may pierce the adjacent tissue 610. Once the microneedle patch 608 pierces the adjacent tissue 610, the ingestible device 600 may delivery a payload (e.g., an API) to the adjacent tissue 610. Additionally, in some embodiments, the body 606 may attach to the adjacent tissue 610 using an adhesive 620 (e.g., a mucoadhesive) to ensure a proper side of the device is oriented towards the tissue prior to deployment of the limb. In some embodiments, the body 606 may have the adhesive 620 pre-applied a desired portion of the body prior to ingestion and the adhesive may be configured to become active when the ingestible device 600 reaches a predetermined position in the GI tract of a subject (e.g., using the methods described in greater detail herein). Thus, the position of the body 606 may remain fixed as the limb 602 deploys such that the microneedle patch 608 comes into contact with and/or pierces the adjacent tissue 610.

As will be appreciated by one of skill in the art, the body 606 need not attach to the adjacent tissue via an adhesive as the body 606 may attach to the adjacent tissue 610 in any suitable manner. For example, in some embodiments, the body 606 may attach to the adjacent tissue 610 using one or more mechanical fasteners such as pins, clamps, barbs, grips, and/or any other suitable type of fastener. Of course, the body 606 may attach to the adjacent tissue 610 in any suitable manner, depending on the application, as the disclosure is not so limited in this regard.

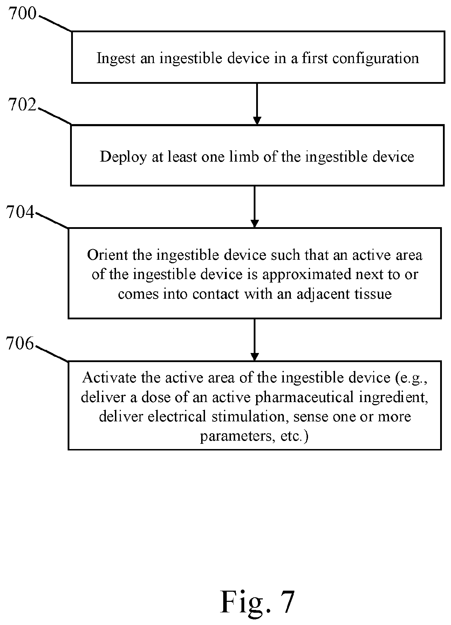

It should also be appreciated that an ingestible device may be administered to and deployed within a subject using any appropriate method. For example, Fig. 7 illustrates a flowchart of an exemplary method for administering and actuating in ingestible device. At step 700, a subject may receive an ingestible device in a first (e.g., undeployed) configuration. The subject may ingest (e.g., orally) the ingestible device in the first configuration. The ingestible device may then travel along the GI tract of the subject until the device reaches a desired location within the GI tract of the subject. Once the ingestible device reaches the desired orientation, at step 702, at least one deployable limb of the ingestible device may be deployed from a first retracted configuration to a second extended configuration in which the at least one limb extends outwards from a body of the ingestible device (e.g., by exposing the at least one deployable limb to a predetermined condition). At step 704, the deployed limb may serve to orient the ingestible device within the GI tract of the subject. Particularly, the ingestible device may be oriented such that an active portion of the ingestible device is oriented towards, and in some instances in contact with, an adjacent supporting tissue that is located vertically below the device relative to a local direction of gravity. Then, at step 706, the active portion of the ingestible device may be activated to perform a function on the adjacent tissue (e.g., deliver a dose of an API, deliver electrical stimulation, sense one or more parameters, etc.).

While the present teachings have been described in conjunction with various embodiments and examples, it is not intended that the present teachings be limited to such embodiments or examples. Accordingly, the foregoing description and drawings are by way of example only.

## Claims

1. An ingestible device (100, 200, 300, 400, 500, 600) comprising:
a body (106, 206, 306, 406, 506, 606);
at least one limb (102, 202, 302, 502, 602) configured to deploy from a first configuration to a second configuration after exposure to a predetermined condition within a gastrointestinal tract of a subject, wherein in the first configuration the at least one limb (102, 202, 302, 502, 602) is wrapped around at least a portion of an outer surface of the body (106, 206, 306, 406, 506, 606), and wherein in the second configuration the limb (102, 202, 302, 502, 602) extends outward from the body (106, 206, 306, 406, 506, 606); and
an active portion (308, 408, 508) of the ingestible device (100, 200, 300, 400, 500, 600) disposed on an exterior portion of the body (106, 206, 306, 406, 506, 606) and/or the at least one limb (102, 202, 302, 502, 602), wherein the at least one limb (102, 202, 302, 502, 602) is configured to orient the body (106, 206, 306, 406, 506, 606) such that the active portion (308, 408, 508) contacts an adjacent tissue surface underlying the body (106, 206, 306, 406, 506, 606) relative to a direction of gravity when the at least one limb (102, 202, 302, 502, 602) is in the second configuration, and wherein the active portion (308, 408, 508) is configured to interact with the adjacent tissue surface.

2. An ingestible device (100, 200, 300, 400, 500, 600) comprising:
a body (106, 206, 306, 406, 506, 606);
a reservoir (414) configured to contain an active pharmaceutical ingredient;
an outlet (408) disposed on an exterior portion of the body (106, 206, 306, 406, 506, 606); and
at least one limb (102, 202, 302, 502, 602) configured to deploy from a first configuration to a second configuration after exposure to a predetermined condition within a gastrointestinal tract of a subject, wherein in the first configuration the at least one limb (102, 202, 302, 502, 602) is wrapped around at least a portion of an outer surface of the body (106, 206, 306, 406, 506, 606), and wherein in the second configuration the limb (102, 202, 302, 502, 602) extends outward from the body (106, 206, 306, 406, 506, 606), wherein the at least one limb (102, 202, 302, 502, 602) is configured to orient the body (106, 206, 306, 406, 506, 606) such that the outlet (408) is oriented towards an adjacent tissue surface underlying the body (106, 206, 306, 406, 506, 606) relative to a direction of gravity when the at least one limb (102, 202, 302, 502, 602) is in the second configuration, and wherein the outlet (408) and reservoir (414) are configured to apply the active pharmaceutical ingredient to the adjacent tissue surface.

3. The ingestible device (100, 200, 300, 400, 500, 600) of any one of claims 1 or 2, wherein the body (106, 206, 306, 406, 506, 606) is an elongated body (106, 206, 306, 406, 506, 606).

4. The ingestible device (100, 200, 300, 400, 500, 600) of any one of claims 1 or 2, wherein the at least one limb (102, 202, 302, 502, 602) is configured to deploy radially outwards from the first configuration to the second configuration in a direction that is angled to a longitudinal axis of the body (106, 206, 306, 406, 506, 606).

5. The ingestible device (100, 200, 300, 400, 500, 600) of any one of claims 1 or 2, wherein the at least one limb (102, 202, 302, 502, 602) is at least partially formed from at least one of a shape-memory material, a carbohydrate, a hydrating starch, a lipid, cellulose, a protein, gelatin, and a polymer bilayer.

6. The ingestible device (100, 200, 300, 400, 500, 600) of any one of claims 1 or 2, wherein the at least one limb (102, 202, 302, 502, 602) includes a first limb (102, 202, 302, 502, 602) and a second limb (104), and wherein at least a portion of the first limb (102, 202, 302, 502, 602) overlaps at least a portion of the second limb (104) in the first configuration.

7. The ingestible device (100, 200, 300, 400, 500, 600) of claim 6, wherein the at least one limb (102, 202, 302, 502, 602) includes a first limb (102, 202, 302, 502, 602) and a second limb (104), and wherein the first limb (102, 202, 302, 502, 602) and the second limb (104) are configured to deploy sequentially.

8. The ingestible device (100, 200, 300, 400, 500, 600) of any one of claims 1 or 2, wherein the at least one limb (102, 202, 302, 502, 602) includes a first limb (102, 202, 302, 502, 602) and a second limb (104), and wherein the first limb (102, 202, 302, 502, 602) is offset from the second limb (104) in a direction parallel to a longitudinal axis of the ingestible device (100, 200, 300, 400, 500, 600).

9. The ingestible device (100, 200, 300,1 400, 500, 600) of any one of claims 1 or 2, wherein the at least one limb (102, 202, 302, 502, 602) is configured to roll the body (106, 206, 306, 406, 506, 606) across the adjacent tissue surface as the at least one limb (102, 202, 302, 502, 602) deploys from the first configuration to the second configuration.

10. The ingestible device (100, 200, 300, 400, 500, 600) of claim 1, wherein the active portion (308, 408, 508) comprises at least one selected from the group of a coating of an active pharmaceutical ingredient disposed on an exterior surface of the body (106, 206, 306, 406, 506, 606), a jet outlet (408), an injection needle outlet (408), a sensor, one or more electrodes, one or more microneedle patches, an adhesive, a mechanical fastener, and one or more biopsy tools.

11. The ingestible device (100, 200, 300, 400, 500, 600) of claim 1, wherein the active portion (308, 408, 508) includes at least one sensor integrated with the at least one limb (102, 202, 302, 502, 602), and wherein the at least one sensor is configured to sense one or more parameters of the adjacent tissue surface.

12. The ingestible device (100, 200, 300, 400, 500, 600) of claim 2, wherein in the second configuration, the outlet (408) is in contact with the adjacent tissue surface.

13. The ingestible device (100, 200, 300, 400, 500, 600) of claim 2, wherein the ingestible device (100, 200, 300, 400, 500, 600) includes a second portion disposed on the exterior portion of the body (106, 206, 306, 406, 506, 606) remotely from the outlet (408), wherein, in the second configuration, the at least one limb (102, 202, 302, 502, 602) is configured to cooperate with the adjacent tissue surface underlying the body (106, 206, 306, 406, 506, 606) to provide spacing between the second portion and the tissue surface, thereby tilting the body (106, 206, 306, 406, 506, 606) to orient the outlet (408) towards the adjacent tissue surface.

14. The ingestible device (100, 200, 300, 400, 500, 600) of claim 2, wherein the ingestible device (100, 200, 300, 400, 500, 600) includes an active portion (308, 408, 508) having at least one sensor integrated with the at least one limb (102, 202, 302, 502, 602), and wherein the at least one sensor is configured to sense one or more parameters of the adjacent tissue surface.

## Patentansprüche

1. Einnehmbare Vorrichtung (100, 200, 300, 400, 500, 600), umfassend:
einen Körper (106, 206, 306, 406, 506, 606);
mindestens ein Glied (102, 202, 302, 502, 602), das ausgebildet ist, um sich nach Aussetzung gegenüber einer vorbestimmten Bedingung innerhalb eines Magen-Darm-Trakts eines Subjekts aus einer ersten Konfiguration in eine zweite Konfiguration zu entfalten, wobei in der ersten Konfiguration das mindestens eine Glied (102, 202, 302, 502, 602) um mindestens einen Abschnitt einer Außenoberfläche des Körpers (106, 206, 306, 406, 506, 606) gewickelt ist, und wobei sich in der zweiten Konfiguration das Glied (102, 202, 302, 502, 602) von dem Körper (106, 206, 306, 406, 506, 606) nach außen erstreckt; und
einen aktiven Abschnitt (308, 408, 508) der einnehmbaren Vorrichtung (100, 200, 300, 400, 500, 600), der an einem äußeren Abschnitt des Körpers (106, 206, 306, 406, 506, 606) und/oder dem mindestens einen Glied (102, 202, 302, 502, 602) angeordnet ist, wobei das mindestens eine Glied (102, 202, 302, 502, 602) ausgebildet ist, um den Körper (106, 206, 306, 406, 506, 606) auszurichten, sodass der aktive Abschnitt (308, 408, 508) mit einer benachbarten Gewebeoberfläche in Kontakt tritt, die unter dem Körper (106, 206, 306, 406, 506, 606) liegend relativ zu einer Schwerkraftrichtung ist, wenn das mindestens eine Glied (102, 202, 302, 502, 602) in der zweiten Konfiguration ist, und wobei der aktive Abschnitt (308, 408, 508) ausgebildet ist, um mit der benachbarten Gewebeoberfläche zusammenzuwirken.

2. Einnehmbare Vorrichtung (100, 200, 300, 400, 500, 600), umfassend:
einen Körper (106, 206, 306, 406, 506, 606);
ein Reservoir (414), das ausgebildet ist, um einen pharmazeutischen Wirkstoff zu enthalten;
einen Auslass (408), der an einem äußeren Abschnitt des Körpers (106, 206, 306, 406, 506, 606) angeordnet ist; und
mindestens ein Glied (102, 202, 302, 502, 602), das ausgebildet ist, um sich nach Aussetzung gegenüber einer vorbestimmten Bedingung innerhalb eines Magen-Darm-Trakts eines Subjekts aus einer ersten Konfiguration in eine zweite Konfiguration zu entfalten, wobei in der ersten Konfiguration das mindestens eine Glied (102, 202, 302, 502, 602) um mindestens einen Abschnitt einer Außenoberfläche des Körpers (106, 206, 306, 406, 506, 606) gewickelt ist, und wobei sich in der zweiten Konfiguration das Glied (102, 202, 302, 502, 602) von dem Körper (106, 206, 306, 406, 506, 606) nach außen erstreckt, wobei das mindestens eine Glied (102, 202, 302, 502, 602) ausgebildet ist, um den Körper (106, 206, 306, 406, 506, 606) auszurichten, sodass der Auslass (408) in Richtung einer benachbarten Gewebeoberfläche ausgerichtet ist, die unter dem Körper (106, 206, 306, 406, 506, 606) liegend relativ zu einer Schwerkraftrichtung ist, wenn das mindestens eine Glied (102, 202, 302, 502, 602) in der zweiten Konfiguration ist, und wobei der Auslass (408) und das Reservoir (414) ausgebildet sind, um den pharmazeutischen Wirkstoff auf die benachbarte Gewebeoberfläche aufzubringen.

3. Einnehmbare Vorrichtung (100, 200, 300, 400, 500, 600) nach einem der Ansprüche 1 oder 2, wobei der Körper (106, 206, 306, 406, 506, 606) ein länglicher Körper (106, 206, 306, 406, 506, 606) ist.

4. Einnehmbare Vorrichtung (100, 200, 300, 400, 500, 600) nach einem der Ansprüche 1 oder 2, wobei das mindestens eine Glied (102, 202, 302, 502, 602) ausgebildet ist, um sich radial nach außen aus der ersten Konfiguration in die zweite Konfiguration in einer Richtung zu entfalten, die zu einer Längsachse des Körpers (106, 206, 306, 406, 506, 606) abgewinkelt ist.

5. Einnehmbare Vorrichtung (100, 200, 300, 400, 500, 600) nach einem der Ansprüche 1 oder 2, wobei das mindestens eine Glied (102, 202, 302, 502, 602) mindestens teilweise aus mindestens einem von einem Formgedächtnismaterial, einem Kohlenhydrat, einer hydratisierenden Stärke, einem Lipid, Cellulose, einem Protein, Gelatine und einer Polymerdoppelschicht gebildet ist.

6. Einnehmbare Vorrichtung (100, 200, 300, 400, 500, 600) nach einem der Ansprüche 1 oder 2, wobei das mindestens eine Glied (102, 202, 302, 502, 602) ein erstes Glied (102, 202, 302, 502, 602) und ein zweites Glied (104) einschließt, und wobei mindestens ein Abschnitt des ersten Glieds (102, 202, 302, 502, 602) mindestens einen Abschnitt des zweiten Glieds (104) in der ersten Konfiguration überlappt.

7. Einnehmbare Vorrichtung (100, 200, 300, 400, 500, 600) nach Anspruch 6, wobei das mindestens eine Glied (102, 202, 302, 502, 602) ein erstes Glied (102, 202, 302, 502, 602) und ein zweites Glied (104) einschließt, und wobei das erste Glied (102, 202, 302, 502, 602) und das zweite Glied (104) ausgebildet sind, um sich nacheinander zu entfalten.

8. Einnehmbare Vorrichtung (100, 200, 300, 400, 500, 600) nach einem der Ansprüche 1 oder 2, wobei das mindestens eine Glied (102, 202, 302, 502, 602) ein erstes Glied (102, 202, 302, 502, 602) und ein zweites Glied (104) einschließt, und wobei das erste Glied (102, 202, 302, 502, 602) gegenüber dem zweiten Glied (104) in einer zu einer Längsachse der einnehmbaren Vorrichtung (100, 200, 300, 400, 500, 600) parallelen Richtung versetzt ist.

9. Einnehmbare Vorrichtung (100, 200, 300,1 400, 500, 600) nach einem der Ansprüche 1 oder 2, wobei das mindestens eine Glied (102, 202, 302, 502, 602) ausgebildet ist, um den Körper (106, 206, 306, 406, 506, 606) über die benachbarte Gewebeoberfläche zu rollen, wenn sich das mindestens eine Glied (102, 202, 302, 502, 602) aus der ersten Konfiguration in die zweite Konfiguration entfaltet.

10. Einnehmbare Vorrichtung (100, 200, 300, 400, 500, 600) nach Anspruch 1, wobei der aktive Abschnitt (308, 408, 508) mindestens eines, ausgewählt aus der Gruppe einer Beschichtung eines pharmazeutischen Wirkstoffs, auf einer Außenoberfläche des Körpers (106, 206, 306, 406, 506, 606) angeordnet, einen Strahlauslass (408), einen Injektionsnadelauslass (408), einen Sensor, eine oder mehrere Elektroden, ein oder mehrere Mikronadelpflaster, ein Klebemittel, ein mechanisches Befestigungselement und ein oder mehrere Biopsiewerkzeuge umfasst.

11. Einnehmbare Vorrichtung (100, 200, 300, 400, 500, 600) nach Anspruch 1, wobei der aktive Abschnitt (308, 408, 508) mindestens einen Sensor einschließt, der mit dem mindestens einen Glied (102, 202, 302, 502, 602) integriert ist, und wobei der mindestens eine Sensor ausgebildet ist, um einen oder mehrere Parameter der benachbarten Gewebeoberfläche zu erfassen.

12. Einnehmbare Vorrichtung (100, 200, 300, 400, 500, 600) nach Anspruch 2, wobei der Auslass (408) in der zweiten Konfiguration mit der benachbarten Gewebeoberfläche in Kontakt ist.

13. Einnehmbare Vorrichtung (100, 200, 300, 400, 500, 600) nach Anspruch 2, wobei die einnehmbare Vorrichtung (100, 200, 300, 400, 500, 600) einen zweiten Abschnitt einschließt, der an dem äußeren Abschnitt des Körpers (106, 206, 306, 406, 506, 606), von dem Auslass (408) entfernt angeordnet ist, wobei, das mindestens eine Glied (102, 202, 302, 502, 602) in der zweiten Konfiguration ausgebildet ist, um mit der benachbarten Gewebeoberfläche, die unter dem Körper (106, 206, 306, 406, 506, 606) liegend ist, zusammenzuwirken, um einen Zwischenraum zwischen dem zweiten Abschnitt und der Gewebeoberfläche bereitzustellen, wodurch sich der Körper (106, 206, 306, 406, 506, 606) neigt, um den Auslass (408) in Richtung der benachbarten Gewebeoberfläche auszurichten.

14. Einnehmbare Vorrichtung (100, 200, 300, 400, 500, 600) nach Anspruch 2, wobei die einnehmbare Vorrichtung (100, 200, 300, 400, 500, 600) einen aktiven Abschnitt (308, 408, 508) einschließt, der mindestens einen Sensor aufweist, der mit dem mindestens einen Glied (102, 202, 302, 502, 602) integriert ist, und wobei der mindestens eine Sensor ausgebildet ist, um einen oder mehrere Parameter der benachbarten Gewebeoberfläche zu erfassen.

## Revendications

1. Dispositif (100, 200, 300, 400, 500, 600) ingérable comprenant :
un corps (106, 206, 306, 406, 506, 606) ;
au moins un membre (102, 202, 302, 502, 602) configuré pour se déployer depuis une première configuration vers une deuxième configuration après une exposition à une condition prédéterminée à l'intérieur d'un tractus gastro-intestinal d'un sujet, dans lequel dans la première configuration, le au moins un membre (102, 202, 302, 502, 602) est enroulé autour d'au moins une partie d'une surface externe du corps (106, 206, 306, 406, 506, 606), et dans lequel dans la deuxième configuration, le membre (102, 202, 302, 502, 602) s'étend vers l'extérieur à partir du corps (106, 206, 306, 406, 506, 606) ; et
une partie (308, 408, 508) active du dispositif (100, 200, 300, 400, 500, 600) ingérable disposée sur une partie extérieure du corps (106, 206, 306, 406, 506, 606) et/ou le au moins un membre (102, 202, 302, 502, 602), dans lequel le au moins un membre (102, 202, 302, 502, 602) est configuré pour orienter le corps (106, 206, 306, 406, 506, 606) de sorte que la partie (308, 408, 508) active entre en contact avec une surface de tissu adjacente située sous le corps (106, 206, 306, 406, 506, 606) par rapport à une direction de la gravité lorsque le au moins un membre (102, 202, 302, 502, 602) se trouve dans la deuxième configuration, et dans lequel la partie (308, 408, 508) active est configurée pour interagir avec la surface de tissu adjacente.

2. Dispositif (100, 200, 300, 400, 500, 600) ingérable comprenant :
un corps (106, 206, 306, 406, 506, 606) ;
un réservoir (414) configuré pour contenir un principe pharmaceutique actif ;
une sortie (408) disposée sur une partie extérieure du corps (106, 206, 306, 406, 506, 606) ; et
au moins un membre (102, 202, 302, 502, 602) configuré pour se déployer depuis une première configuration vers une deuxième configuration après une exposition à une condition prédéterminée à l'intérieur d'un tractus gastro-intestinal d'un sujet, dans lequel dans la première configuration, le au moins un membre (102, 202, 302, 502, 602) est enroulé autour d'au moins une partie d'une surface externe du corps (106, 206, 306, 406, 506, 606), et dans lequel dans la deuxième configuration, le membre (102, 202, 302, 502, 602) s'étend vers l'extérieur à partir du corps (106, 206, 306, 406, 506, 606), dans lequel le au moins un membre (102, 202, 302, 502, 602) est configuré pour orienter le corps (106, 206, 306, 406, 506, 606) de sorte que la sortie (408) soit orientée vers une surface de tissu adjacente située sous le corps (106, 206, 306, 406, 506, 606) par rapport à une direction de la gravité lorsque le au moins un membre (102, 202, 302, 502, 602) se trouve dans la deuxième configuration, et dans lequel la sortie (408) et le réservoir (414) sont configurés pour appliquer le principe pharmaceutique actif à la surface de tissu adjacente.

3. Dispositif (100, 200, 300, 400, 500, 600) ingérable selon l'une quelconque des revendications 1 ou 2, dans lequel le corps (106, 206, 306, 406, 506, 606) est un corps (106, 206, 306, 406, 506, 606) allongé.

4. Dispositif (100, 200, 300, 400, 500, 600) ingérable selon l'une quelconque des revendications 1 ou 2, dans lequel le au moins un membre (102, 202, 302, 502, 602) est configuré pour se déployer radialement vers l'extérieur depuis la première configuration vers la deuxième configuration dans une direction qui fait un angle par rapport à un axe longitudinal du corps (106, 206, 306, 406, 506, 606).

5. Dispositif (100, 200, 300, 400, 500, 600) ingérable selon l'une quelconque des revendications 1 ou 2, dans lequel le au moins un membre (102, 202, 302, 502, 602) est au moins partiellement formé à partir d'au moins l'un ou l'une parmi un matériau à mémoire de forme, un glucide, un amidon hydratant, un lipide, la cellulose, une protéine, la gélatine, et une bicouche polymère.

6. Dispositif (100, 200, 300, 400, 500, 600) ingérable selon l'une quelconque des revendications 1 ou 2, dans lequel le au moins un membre (102, 202, 302, 502, 602) inclut un premier membre (102, 202, 302, 502, 602) et un deuxième membre (104), et dans lequel au moins une partie du premier membre (102, 202, 302, 502, 602) se chevauche avec au moins une partie du deuxième membre (104) dans la première configuration.

7. Dispositif (100, 200, 300, 400, 500, 600) ingérable selon la revendication 6, dans lequel le au moins un membre (102, 202, 302, 502, 602) inclut un premier membre (102, 202, 302, 502, 602) et un deuxième membre (104), et dans lequel le premier membre (102, 202, 302, 502, 602) et le deuxième membre (104) sont configurés pour se déployer séquentiellement.

8. Dispositif (100, 200, 300, 400, 500, 600) ingérable selon l'une quelconque des revendications 1 ou 2, dans lequel le au moins un membre (102, 202, 302, 502, 602) inclut un premier membre (102, 202, 302, 502, 602) et un deuxième membre (104), et dans lequel le premier membre (102, 202, 302, 502, 602) est décalé par rapport au deuxième membre (104) dans une direction parallèle à un axe longitudinal du dispositif (100, 200, 300, 400, 500, 600) ingérable.

9. Dispositif (100, 200, 300,1 400, 500, 600) ingérable selon l'une quelconque des revendications 1 ou 2, dans lequel le au moins un membre (102, 202, 302, 502, 602) est configuré pour faire rouler le corps (106, 206, 306, 406, 506, 606) sur la surface de tissu adjacente à mesure que le au moins un membre (102, 202, 302, 502, 602) se déploie depuis la première configuration vers la deuxième configuration.

10. Dispositif (100, 200, 300, 400, 500, 600) ingérable selon la revendication 1, dans lequel la partie (308, 408, 508) active comprend au moins un sélectionné dans le groupe constitué d'un revêtement d'un principe pharmaceutique actif disposé sur une surface extérieure du corps (106, 206, 306, 406, 506, 606), d'une sortie (408) de jet, d'une sortie (408) d'aiguille d'injection, d'un capteur, d'une ou de plusieurs électrodes, d'un ou de plusieurs timbres à micro-aiguilles, d'un adhésif, d'un dispositif de fixation mécanique, et d'un ou de plusieurs outils de biopsie.

11. Dispositif (100, 200, 300, 400, 500, 600) ingérable selon la revendication 1, dans lequel la partie (308, 408, 508) active inclut au moins un capteur intégré au au moins un membre (102, 202, 302, 502, 602), et dans lequel le au moins un capteur est configuré pour détecter un ou plusieurs paramètres de la surface de tissu adjacente.

12. Dispositif (100, 200, 300, 400, 500, 600) ingérable selon la revendication 2, dans lequel, dans la deuxième configuration, la sortie (408) se trouve en contact avec la surface de tissu adjacente.

13. Dispositif (100, 200, 300, 400, 500, 600) ingérable selon la revendication 2, dans lequel le dispositif (100, 200, 300, 400, 500, 600) ingérable inclut une deuxième partie disposée sur la partie extérieure du corps (106, 206, 306, 406, 506, 606) à distance de la sortie (408), dans lequel, dans la deuxième configuration, le au moins un membre (102, 202, 302, 502, 602) est configuré pour coopérer avec la surface de tissu adjacente située sous le corps (106, 206, 306, 406, 506, 606) pour fournir un espacement entre la deuxième partie et la surface de tissu, ce qui a pour effet d'incliner le corps (106, 206, 306, 406, 506, 606) pour orienter la sortie (408) vers la surface de tissu adjacente.

14. Dispositif (100, 200, 300, 400, 500, 600) ingérable selon la revendication 2, dans lequel le dispositif (100, 200, 300, 400, 500, 600) ingérable inclut une partie (308, 408, 508) active présentant au moins un capteur intégré au au moins un membre (102, 202, 302, 502, 602), et dans lequel le au moins un capteur est configuré pour détecter un ou plusieurs paramètres de la surface de tissu adjacente.
